(19) Europäisches Patentamt
European Patent Office
Office européen des brevets

(11) EP 1 941 834 A1

(12) EUROPEAN PATENT APPLICATION
published in accordance with Art. 153(4) EPC

(43) Date of publication:
09.07.2008 Bulletin 2008/28

(51) Int Cl.:
A61B 5/1455 (2006.01)    A61B 1/227 (2006.01)
A61B 1/233 (2006.01)

(21) Application number: 06812130.0

(22) Date of filing: 23.10.2006

(86) International application number:
PCT/JP2006/321070

(87) International publication number:
WO 2007/049562 (03.05.2007 Gazette 2007/18)

(84) Designated Contracting States:
DE

(30) Priority: 24.10.2005   JP 2005308156
10.03.2006   JP 2006065363

(71) Applicant: Matsushita Electric Industrial Co., Ltd.
Kadoma-shi
Osaka 571-8501 (JP)

(72) Inventor: UCHIDA, Shinji,
Matsushita Electric Industrial Co., Ltd.
2-1-61, Shiromi,
Chuo-ku,
Osaka 540-6207 (JP)

(74) Representative: TBK-Patent
Bavariaring 4-6
80336 München (DE)

(54) APPARATUS FOR MEASURING BIOLOGICAL COMPONENT CONCENTRATION

(57) To provide a biocomponent concentration measuring device capable of determining which organ in an ear cavity is in condition for a measurement, the biocomponent concentration measuring device is provided with an infrared ray detector 108 for detecting an infrared light irradiated from an organ in an ear cavity 200, and an image sensor 148 for picking up an image of the organ in the ear cavity 200.

FIG. 2

EP 1 941 834 A1

## Description

Technical Field

The present invention relates to a biocomponent concentration measuring device for noninvasively measuring a concentration of a biocomponent (for example, glucose), without taking blood sample.

Background Art

There has been proposed so far, as a bioinformation measurement device, a noninvasive blood sugar meter for measuring an irradiated light from an eardrum and calculating a glucose concentration (for example, see patent document 1, 2, or 3). For example, patent document 1 discloses a noninvasive blood sugar meter that has a mirror with a size that can be put inside an external ear canal; applies a near-infrared ray or a heat ray to the eardrum via that mirror; detects the light reflected at the eardrum; and calculates the glucose concentration from the detected results. Patent document 2 discloses a noninvasive blood sugar meter that has a probe to be inserted in the ear cavity; detects an infrared ray generated in the inner ear and irradiated from the eardrum via the probe while the eardrum and the external ear canal are cooled; and analyzes the detected infrared ray by spectroscopy to obtain a glucose concentration. Patent document 3 discloses a noninvasive blood sugar meter that has a reflection mirror to be inserted in the ear cavity; detects an irradiated light from the eardrum by using the reflection mirror; and analyzes the detected irradiated light by spectroscopy to obtain a glucose concentration.

[Patent Document 1] Japanese Unexamined Patent Application No. Hei 05-506171
[Patent Document 2] Japanese Unexamined Patent Application No. 2002-513604
[Patent Document 3] Japanese Unexamined Patent Application No. 2001-503999

Disclosure of the Invention

Problem to be Solved by the Invention

However, with the above conventional noninvasive blood sugar meter, it was difficult to determine which organ in the ear cavity was being measured, because the direction of the mirror or probe inserted in the ear cavity could not be checked.
Thus, in view of the above conventional problem, the present invention aims to provide a biocomponent concentration measuring device capable of determining which organ in the ear cavity is in condition for the measurement.

Means for Solving the Problem

To solve the above conventional problem, a biocomponent concentration measuring device of the present invention includes an infrared ray detector for detecting an infrared light irradiated from an organ in the ear cavity; an image sensor for picking up an image of the organ in the ear cavity; and a biocomponent concentration computing unit for converting an output of the infrared ray detector to a biocomponent concentration.

Effects of the Invention

Based on a biocomponent concentration measuring device of the present invention, it is possible to know which organ in the ear cavity is in condition for the measurement.

Brief Description of the Drawings

[FIG. 1] A perspective view illustrating an exterior of a biocomponent concentration measuring device in one embodiment of the present invention.
[FIG. 2] A diagram illustrating a configuration of the biocomponent concentration measuring device.
[FIG. 3] A perspective view illustrating an optical filter wheel in the biocomponent concentration measuring device.
[FIG. 4] A diagram illustrating an image upon observing the ear cavity by using an image sensor for picking up a black and white image.
[FIG. 5] A diagram illustrating a result of a binary image processing on the image of FIG. 4.

[FIG. 6] A diagram illustrating a configuration of a biocomponent concentration measuring device in another embodiment of the present invention.
[FIG. 7] A diagram illustrating a configuration of a biocomponent concentration measuring device in still another embodiment of the present invention.
[FIG. 8] A diagram illustrating a configuration of a biocomponent concentration measuring device in still another embodiment of the present invention.
[FIG. 9] A perspective view illustrating an exterior of a biocomponent concentration measuring device in still another embodiment of the present invention.
[FIG. 10] A diagram illustrating a configuration of the biocomponent concentration measuring device.
[FIG. 11] A diagram illustrating a configuration of a biocomponent concentration measuring device in a variation of the embodiment.
[FIG. 12] A graph illustrating effects of the external ear canal on radiance.

Best Mode for Carrying Out the Invention

A biocomponent concentration measuring device of the present invention comprises:

an infrared ray detector for detecting an infrared light irradiated from an organ in the ear cavity;
an image sensor for picking up an image of the organ in the ear cavity; and
a biocomponent concentration computing unit for converting an output of the infrared ray detector to a biocomponent concentration.

Based on such a configuration, by detecting an infrared light irradiated from the organ in the ear cavity with the infrared ray detector, upon measuring the biocomponent concentration, the infrared ray detector can determine which organ in the ear cavity is in condition for the detection of the infrared light irradiated therefrom: therefore, it is possible to know which organ in the ear cavity can be measured by the biocomponent concentration measuring device.

In the present invention, the image sensor includes, for example, CMOS and CCD.
The infrared ray detector includes those detectors that can detect a light with a wavelength in the infrared range. For example, pyroelectric sensor, thermopile, bolometer, HgCdTe (MCT) detector, and Golay cell may be used.

For the biocomponent concentration computing unit, for example, a microcomputer such as CPU (Central Processing Unit) may be used.
The biocomponent concentration measuring device of the present invention preferably further comprises a light source for emitting a light for lighting up inside the ear cavity, and the image sensor preferably picks up an image of a light emitted from the light source and reflected from the organ in the ear cavity.

For the light source in the present invention, for example, visible laser such as red laser, and white LED may be used. Among these light sources, white LED is preferable in that the heat generation upon the emission is less compared with halogen lamp, and therefore effects on temperatures of the eardrum and the external ear canal are less. Instead of the light source emitting the visible light, a light source emitting near-infrared light may also be used.
The biocomponent concentration measuring device of the present invention preferably further comprises a spectroscopic element for dispersing the infrared light.
For the spectroscopic element, any spectroscopic element may be used as long as it can disperse the infrared light by wavelength: for example, an optical filter, a spectroscopic prism, a Michelson interferometer, and a diffraction grating, which transmit infrared light in a specific wavelength range.

The biocomponent concentration measuring device of the present invention may further comprises an insertion probe to be inserted in the ear cavity, and the insertion probe may have functions of guiding the infrared light irradiated from the organ in the ear cavity to the infrared ray detector.
Any insertion probe may be used in the present invention, as long as it can guide infrared ray: for example, a hollow pipe, and optical fiber that transmits infrared ray may be used. When the hollow pipe is to be used, a gold layer is preferably provided at the inner surface of the hollow pipe. The gold layer may be formed by gold-plating, or by vapor depositing gold at the inner surface of the hollow pipe.

The biocomponent concentration measuring device of the present invention may further comprises a light-splitting element that transmits one of the light that was reflected from the organ in the ear cavity and the infrared light that was irradiated from the organ in the ear cavity; and reflects the other of the light that was reflected from the organ in the ear cavity and the infrared light that was irradiated from the organ in the ear cavity.
The spectroscopic element may be disposed between the infrared ray detector and the light-splitting element.

In the present invention, may be used for the light-splitting element are, for example, a beam splitter that transmits visible light and near-infrared light, and reflects infrared light, among visible light, near-infrared light, and infrared light; and a beam splitter that transmits infrared light, and reflects visible light and near-infrared light, among visible light, near-infrared light, and infrared light. In the case when visible light and near-infrared light are to be reflected and infrared ray is to be transmitted, for the beam splitter material, for example, ZnSe, CaF$_2$, Si, and Ge may be used. Also, on a resin that is transparent to infrared ray, a layer comprising aluminum or gold with a thickness of several nanometers may be provided and used. For the resin transparent to infrared ray, for example, polycarbonate, polypropylene, and polystyrene may be mentioned.

The biocomponent concentration measuring device of the present invention preferably further comprises a captured-image information detecting unit for detecting information on the captured-image of the eardrum from the information on the captured-image obtained by the image sensor.

Such a configuration enables automatic determination of whether or not the eardrum is included in the organ irradiating the infrared light detected by the infrared ray detector.

For the captured-image information detecting unit, for example, an image processing LSI and a microcomputer may be used. For the microcomputer, for example, CPU (Central Processing Unit) may be mentioned.

For the method for the captured-image information detecting unit to detect the information on the captured-image of the eardrum from the information on the captured-image obtained by the image sensor, may be mentioned is a method in which image processing of the information on the captured-image obtained by the image sensor is carried out, and the differences in the image brightness are used to recognize the eardrum image.

When the image sensor includes a plurality of pixels and the output signal outputted from each of the plurality of pixels forms the information on the captured-image, the captured-image information detecting unit may detect, among the output signals, the output signals equal to or smaller than the preset reference value as the information on the captured-image of the eardrum.

The biocomponent concentration computing unit preferably corrects the biocomponent concentration based on the amount of information on the captured-image of the eardrum detected by the captured-image information detecting unit.

Further preferably, the captured-image information detecting unit calculates the proportion of the information on the captured-image of the eardrum in the information on the captured-image obtained by the image sensor as the amount of the information on the captured-image of the eardrum, and the biocomponent concentration computing unit corrects the biocomponent concentration by using the proportion. The intensity of the infrared light irradiated from a living subject depends on the area of the portion irradiating the infrared light. Therefore, even though there are variations in the area of the image of the eardrum captured by the image sensor, such a correction enables a decrease in the variations in the measurement results, and therefore enables a highly accurate measurement.

The biocomponent concentration measuring device of the present invention may further comprise a sound source for outputting a sound while the captured-image information detecting unit is detecting the information on the captured-image of the eardrum.

At least one of volume, frequency, and output interval of the sound outputted from the sound source preferably changes according to the amount of the information on the captured-image of the eardrum detected by the captured-image information detecting unit. Based on such a configuration, a user can be notified of the change in the amount of the information on the captured-image of the eardrum detected by the captured-image information detecting unit by the change in the sound outputted from the sound source.

The biocomponent concentration measuring device of the present invention preferably further comprises a warning output unit for outputting warning when the amount of the information on the captured-image of the eardrum detected by the captured-image information detecting unit is equal to or below the threshold. Based on such a configuration, the user can be notified of an inappropriate position of the biocomponent concentration measuring device.

For the warning output unit, a display for showing the warning, a speaker for outputting a warning with a sound, and a buzzer for producing a warning sound may be mentioned.

The biocomponent concentration measuring device of the present invention preferably further comprises an optical path control element for controlling an optical path so that the infrared light irradiated from the eardrum is selectively transmitted based on the information on the captured-image of the eardrum detected by the captured-image information detecting unit.

Based on such a configuration, the infrared light irradiated from the eardrum is transmitted through the optical path control element and reaches the infrared ray detector, and the infrared light irradiated from external ear canal is blocked by the optical path control element and does not reach the infrared ray detector; therefore, effects from the external ear canal can be removed, and a highly accurate measurement can be conducted.

The optical path control element is preferably provided between the infrared ray detector and the light-splitting element. Based on such a configuration, since there is no optical path control element on the optical path of the visible light and the near-infrared light that reach the image sensor, the light with a wavelength necessary for picking up an image is not attenuated by the optical path control element, and therefore the image sensor can pickup well an image of the organ in the ear cavity. For the optical path control element, a liquid crystal shutter and a mechanical shutter may be used.

The liquid crystal shutter preferably includes, for example, TFT (Thin Film Transistor), and is capable of allowing and blocking a transmittance of an infrared light in a particular region by controlling with the TFT.

May be used for the mechanical shutter is, for example, a digital mirror device (hereinafter referred to as DMD), i.e., a known technique using MEMS technique, in which micro mirror faces are arranged in a flat plane. The DMD may be made by using known MEMS (Micro Electro Mechanical System). Each of the micromirrors can be controlled to be in two conditions, i.e., ON and OFF, by driving the electrode provided below the mirror face. When the micromirror is ON, the infrared light irradiated from the eardrum is reflected and projected to the infrared ray detector, and when the micromirror is OFF, infrared light is reflected to the absorber provided in the DMD and is not projected to the infrared ray detector. Therefore, by driving each of the micromirrors individually, the projection of the infrared light can be controlled by minute region.

The biocomponent concentration measuring device of the present invention may further include a display for displaying the information on the captured-image obtained by the image sensor; and an instruction reception unit for receiving the instruction on a start of the biocomponent concentration measurement, and the display may be disposed at a position where the user can see the information on the captured-image shown on the display while the insertion probe is being inserted in the ear cavity of the user.

Based on such a configuration, the user can enter the instruction on measurement start via the instruction reception unit after confirming that the eardrum is shown on the display while the insertion probe of the biocomponent concentration measuring device being inserted in the ear cavity of the user, the effects from the external ear canal can be decreased, and highly accurate measurement can be conducted.

For the instruction reception unit, for example, switch, and button may be mentioned.

The biocomponent concentration measuring device of the present invention may further include a memory unit for storing correlational data showing correlations between the output signal of the infrared ray detector and the biocomponent concentration; a display unit for displaying the biocomponent concentration converted by the biocomponent concentration computing unit; and a power source for supplying electric power for the biocomponent concentration measuring device to operate.

The biocomponent concentration computing unit may convert the output signal of the infrared ray detector into the biocomponent concentration by reading the correlational data from the memory unit and referring to it.

The correlational data showing the correlation between the output signal of the infrared ray detector and the biocomponent concentration can be obtained, for example, by measuring the output signal of the infrared ray detector on a testee with a known biocomponent concentration (for example, a blood-sugar level), and analyzing the obtained correlations between the output signal of the infrared ray detector and the biocomponent concentration.

In the present invention, for the memory unit, for example, a memory such as RAM and ROM may be used.

For the display unit, for example, a display of liquid crystal may be used.

For the power source, for example, a battery may be used.

The biocomponent concentration to be measured by the biocomponent concentration measuring device of the present invention includes, a glucose concentration (a blood-sugar level), a hemoglobin concentration, a cholesterol concentration, and a neutral fat concentration. By measuring the infrared light irradiated from a living subject, bioinformation, for example, a blood-sugar level can be measured. Radiant energy W of the infrared irradiated light from the living subject is represented by the mathematical expression below.

[Mathematical Expression 1]

$$W = s \int_{\lambda_1}^{\lambda_2} \varepsilon(\lambda) \cdot W_0(\mathrm{T}, \lambda) d\lambda \quad (W)$$

[Mathematical Expression 2]

$$W_0(\lambda, T) = 2hc^2 \left\{ \lambda^5 \cdot \left[ \exp(hc/\lambda kT) - 1 \right] \right\}^{-1} \left( W / cm^2 \cdot \mu m \right)$$

The respective symbols in the above expressions represent the following.
W: Radiant Energy of Infrared Irradiated Light from Living Subject
$\varepsilon(\lambda)$ : Emissivity of Living Subject at Wavelength $\lambda$
$W_0(\lambda, T)$: Black Body Radiation Intensity Density at wavelength $\lambda$, temperature T
h: Plank's Constant (h=6.625 $\times$ 10$^{-34}$ (W$\cdot$ S$^2$))
c: Light Velocity (c=2.998 $\times$ 10$^{10}$(cm/s))
$\lambda_1$, $\lambda_2$: Wavelength ($\mu$m) of Infrared Irradiated Light from Living Subject
T: Temperature (K) of Living Subject
S: Detected Area (cm$^2$)
k: Boltzmann constant
As is clear from Mathematical Expression 1, when detected area S is constant, radiant energy W of the infrared irradiated light from a living subject depends on emissivity $\varepsilon(\lambda)$ of the living subject at wavelength $\lambda$. Based on Kirchhoff's law of radiation, emissivity equals absorptivity at the same temperature and the same wavelength.
[Mathematical Expression 3]

$$\varepsilon(\lambda) = \alpha(\lambda)$$

In Mathematical Expression 3, $\alpha(\lambda)$ represents the absorptivity of a living subject at wavelength $\lambda$. Therefore, upon considering the emissivity, the absorptivity may be considered. Based on the law of conservation of energy, absorptivity, transmittance, and reflectivity satisfy the following relation.
[Mathematical Expression 4]

$$\alpha(\lambda) + r(\lambda) + t(\lambda) = 1$$

The respective symbols in the above expression represent the following.
r ($\lambda$): Reflectivity of Living Subject at Wavelength $\lambda$.
t ($\lambda$): Transmittance of Living Subject at Wavelength $\lambda$
Therefore, the emissivity can be expressed as the following, by using the transmittance and the reflectivity.
[Mathematical Expression 5]

$$\varepsilon(\lambda) = \alpha(\lambda) = 1 - r(\lambda) - t(\lambda)$$

The transmittance is expressed as the ratio of the amount of incident light to the amount of the light transmitted through the measurement subject. The amount of incident light and the amount of the light transmitted through the measurement subject are shown with Lambert-Beer law.

[Mathematical Expression 6]

$$I_t(\lambda) = I_0(\lambda) \exp\left(-\frac{4\pi k(\lambda)}{\lambda} d\right)$$

The respective symbols in the above expression represent the following.

$I_t$: Amount of Transmitted Light

$I_0$: Amount of Incident Light

d: Thickness of Living Subject

$k(\lambda)$: Extinction Coefficient of Living Subject at Wavelength $\lambda$. The extinction coefficient of a living subject is a coefficient showing the light absorption by the living subject.

Therefore, the transmittance can be expressed as the following.

[Mathematical Expression 7]

$$t(\lambda) = \exp\left(-\frac{4\pi k(\lambda)}{\lambda} d\right)$$

The reflectivity is described next. Regarding reflectivity, an average of the reflectivities of all directions has to be calculated. However, for simplification, the reflectivity in normal incidence is considered. The reflectivity in normal incidence is expressed as the following, setting the refractive index of air as 1.

[Mathematical Expression 8]

$$r(\lambda) = \frac{(n(\lambda)-1)^2 + k^2(\lambda)}{(n(\lambda)+1)^2 + k^2(\lambda)}$$

In the expression, $n(\lambda)$ shows the refractive index of a living subject at wavelength $\lambda$.

From the above, the emissivity is expressed as the following.

$$\varepsilon(\lambda) = 1 - r(\lambda) - t(\lambda) = 1 - \frac{(n(\lambda)-1)^2 + k(\lambda)^2}{(n(\lambda)+1)^2 + k(\lambda)^2} - \exp\left(-\frac{4\pi k(\lambda)}{\lambda} d\right)$$

When the concentration of a component in a living subject changes, the refractive index and the extinction coefficient of the living subject change. The reflectivity is low, usually about 0.03 in the infrared range, and as can be seen from Mathematical Expression 8, it is not much dependent on the refractive index and the extinction coefficient. Therefore, even the refractive index and the extinction coefficient change with changes in the concentration of a component in a living subject, the changes in the reflectivity is small.

On the other hand, as is clear from Mathematical Expression 7, the transmittance depends heavily on the extinction coefficient. Therefore, when the extinction coefficient of a living subject, i.e., the degree of light absorption by the living subject, changes by changes in the concentration of a component in the living subject, the transmittance changes.

The above clarifies that the radiant energy of an infrared irradiated light from a living subject depends on the concentration of a component in the living subject. Therefore, a concentration of a component in a living subject can be determined from the radiant energy intensity of the infrared irradiated light from the living subject.

Also, as is clear from Mathematical Expression 7, the transmittance is dependent on the thickness of a living subject. The smaller the thickness of the living subject, the larger the degree of the change in the transmittance relative to the change in the extinction coefficient of the living subject, and therefore changes in the component concentration in a living subject can be easily detected. Since eardrums have a small thickness of about 60 to 100 $\mu$m, it is suitable for a concentration measurement of a component in a living subject using infrared irradiated light.

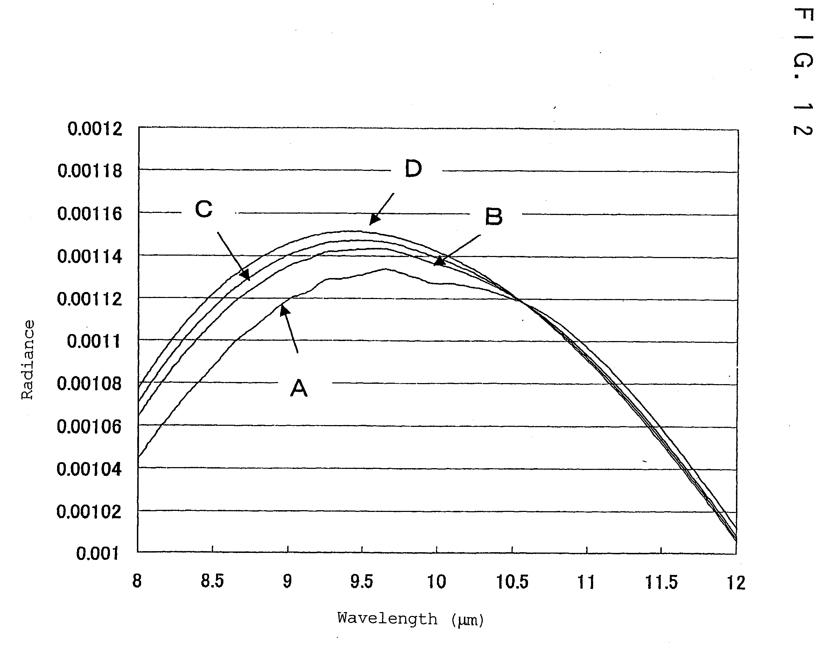

FIG. 12 is a graph illustrating changes in the radiance spectrum by the proportions of the included signal of the infrared light irradiated from the external ear canal, in the case when the signal of the infrared light irradiated from the external ear canal is superimposed on the signal of the infrared light irradiated from an aqueous solution of glucose, i.e., a component in a living subject.

The radiance spectrum of the aqueous solution of glucose was determined by computer simulation by setting the temperature to 37 degrees (A in FIG. 12). The radiance spectrum of the external ear canal is measured with the external ear canal temperature of 36.5 degrees (D in FIG. 12). Also, the radiance spectrum of the case when the signal of the infrared light irradiated from the aqueous solution of glucose is superimposed by the signal of the infrared light irradiated from the external ear canal with a ratio of 3/5 (B in FIG. 12), and the radiance spectrum of the case when the signal of the infrared light irradiated from the aqueous solution of glucose is superimposed by the signal of the infrared light irradiated from the external ear canal with a ratio of 4/5 (C in FIG. 12) were obtained by computer simulation.

FIG. 12 shows that in the case when the signal of the infrared light irradiated from the external ear canal is not included, that is, in the case when only the signal of the infrared light irradiated from the aqueous solution of glucose is included, the radiance spectrum has a peak unique to glucose in the proximity to 9.6 $\mu$m.

FIG. 12 also shows that compared with the signal only consisting of the infrared light irradiated from the aqueous solution of glucose, as the proportion of the signal of the infrared light irradiated from the external ear canal relative to the signal in total increases to 3/5 and 4/5, the intensity of the peak unique to glucose is less obvious. In the radiance spectrum, in the case when the signal only consists of the infrared light irradiated from the external ear canal, the peak unique to glucose almost disappeared.

Therefore, when the signal of the infrared light irradiated from the external ear canal is included in the signal to be measured, it affects greatly to the glucose measurement.

In the following, Embodiments of the present invention are described in detail by referring to the drawings.

Embodiment 1

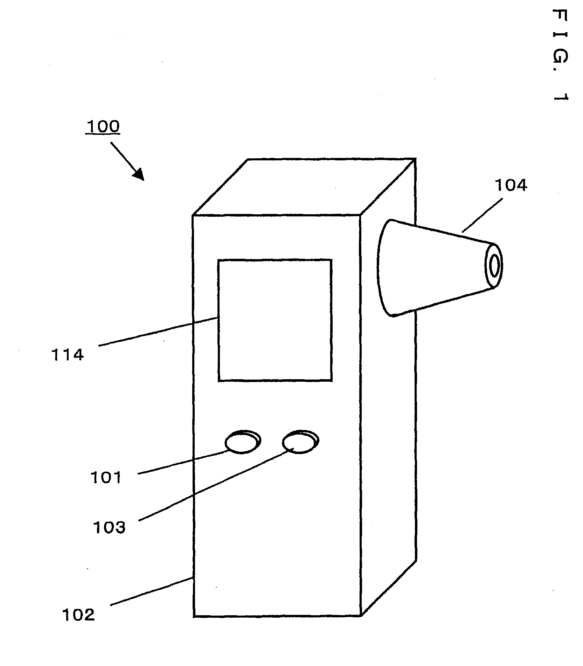

FIG. 1 is a perspective view illustrating an exterior of a biocomponent concentration measuring device 100 of Embodiment 1.

The biocomponent concentration measuring device 100 includes a main body 102, and an insertion probe 104 provided on the side face of the main body 102. The main body 102 includes a display 114 for displaying the measurement results of the concentration of a component in a living subject, a power source switch 101 for ON/OFF of a power source of the biocomponent concentration measuring device 100, and a measurement start switch 103 for starting the measurement. The display 114 corresponds to the display unit in the present invention.

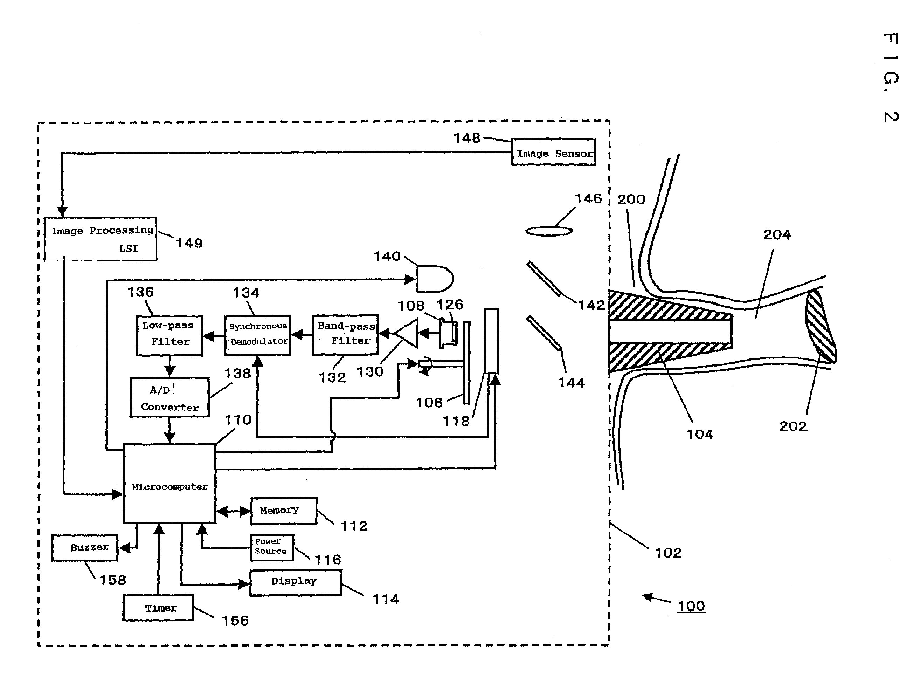

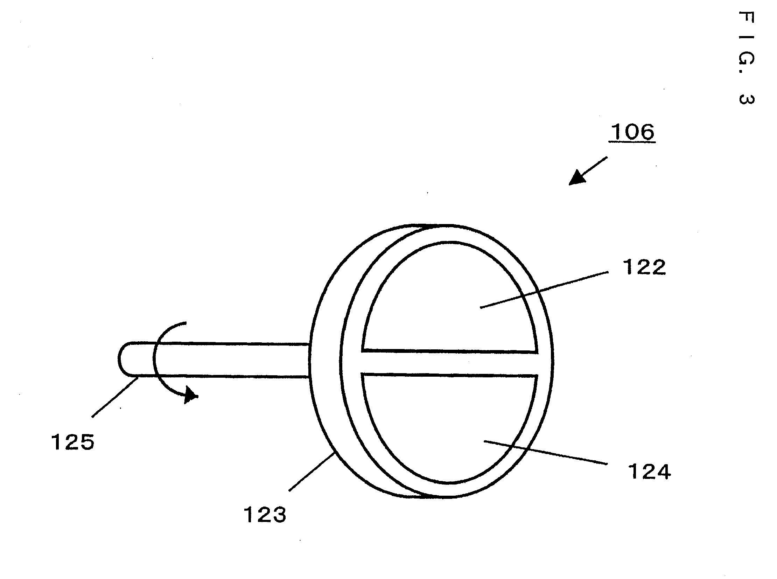

Next, an internal structure of the main body of the biocomponent concentration measuring device 100 is described by using FIG. 2 and FIG. 3. FIG. 2 is a diagram showing a configuration of a biocomponent concentration measuring device 100, and FIG. 3 is a perspective illustration of an optical filter wheel 106 of the biocomponent concentration measuring device 100 in Embodiment 1.

In the main body of the biocomponent concentration measuring device 100, a chopper 118, an optical filter wheel 106, an infrared ray detector 108, a preliminary amplifier 130, a band-pass filter 132, a synchronous demodulator 134, a low-pass filter 136, an analog/digital converter (hereinafter referred to as A/D converter) 138, a microcomputer 110, a memory 112, a display 114, a power source 116, a light source 140, a first beam splitter 142, a second beam splitter 144, a light collection lens 146, an image sensor 148, an image processing LSI 149, a timer 156, and a buzzer 158 are provided. The microcomputer 110 corresponds to the captured-image information detecting unit and the biocomponent concentration computing unit of the present invention.

The power source 116 supplies AC or DC electric power to the microcomputer 110. For the power source 116, a battery is preferably used.

The chopper 118 functions to chop the infrared light, that was irradiated from the eardrum 202, guided into the main body 102 by the insertion probe 104, and transmitted through the second beam splitter 144, and to convert the infrared light into a high-frequency infrared ray signal. The operations of the chopper 118 are controlled by control signals from the microcomputer 110. The infrared light chopped by the chopper 118 reaches the optical filter wheel 106.

As shown in FIG. 3, in the optical filter wheel 106, a first optical filter 122 and a second optical filter 124 are put in a ring 123. In an example shown in FIG. 3, a disk-like member is formed by putting the first optical filter 122 and the second optical filter 124, both being semi-circle, in the ring 123, and a shaft 125 is provided at the center of the disk-like member.

By rotating this shaft 125 following the arrow in FIG. 3, the optical filter for the infrared light chopped by the chopper 118 to pass through can be switched between the first optical filter 122 and the second optical filter 124. The rotation of the shaft 125 is controlled by the control signal from the microcomputer 110.

The rotation of the shaft 125 is preferably synchronized with the rotation of the chopper 118, and controlled so that the shaft 125 is rotated to 180 degrees while the chopper 118 is closed. With such an arrangement, when the chopper 118 is opened next time, the optical filter for the infrared light chopped by the chopper 118 to pass through can be switched to another optical filter.

The optical filter wheel 106 corresponds to the spectroscopic element in the present invention.

The optical filter may be made by any known methods without particular limitation. For example, a vapor deposition method, an ion beam sputtering method, and a CVD method may be used. The optical filter may be made, for example, by the ion beam sputtering method, making a layer of Ge, ZnS, $MgF_2$, and PbTe on a base plate using Si or Ge.

By adjusting the thickness of the each film, the order of the films to be layered, and the times to be layered to control the light interference in the multilayered thin film, an optical filter with desired wavelength characteristics can be made.

The infrared light transmitted through the first optical filter 122 or the second optical filter 124 reaches an infrared ray detector 108 including a detection region 126. The infrared light that reached the infrared ray detector 108 enters the detection region 126, and is converted to an electric signal corresponding to the intensities of the infrared light entered.

The electric signal outputted from the infrared ray detector 108 is amplified by the preliminary amplifier 130. In the amplified electric signal, the signal outside the center frequency, i.e., the frequency band of the chopping, is removed by the band-pass filter 132. Based on this, noise caused by statistical fluctuation such as thermal noise can be minimized.

The electric signal filtered by the band-pass filter 132 is demodulated to DC signal by the synchronous demodulator 134, by synchronizing and integrating the chopping frequency of the chopper 118 and the electric signal filtered by the band-pass filter 132.

In the electric signal demodulated by the synchronous demodulator 134, the signal in the low-frequency band is removed by the low-pass filter 136.

Based on this, noise is further removed.

The electric signal filtered by the low-pass filter 136 is converted to a digital signal by the A/D converter 138, and then inputted to the microcomputer 110. The electric signal from the infrared detector 108 can be identified by using the control signal for the shaft 125 as a trigger, i.e., it can be identified from which optical filter the infrared light was transmitted through. The electric signal can be identified to which optical filter it corresponds, based on an interval of the output of the control signal for the shaft 125 from the microcomputer to the next output of the control signal for the shaft. By adding the respective electric signals for each of the optical filters and then calculating the average in the memory 112, noise is further reduced. Therefore, such an addition is preferably carried out.

The memory 112 stores a plurality of correlational data, being different according to the level of the proportion of the eardrum region in the image picked up by the image sensor 148, and showing correlations between the concentration of a component of a living subject, and the electric signal corresponding to the intensity of the infrared light transmitted through the first optical filter 122 and the electric signal corresponding to the intensity of the infrared light transmitted through the second optical filter 124. The plurality of correlational data being different according to the level of the eardrum region proportion can be obtained, for example, by changing the direction of the insertion probe 104 upon measurement.

The microcomputer 110 reads out the correlational data corresponding to the level of the proportion of the eardrum region in the image picked up by the image sensor 148, which is obtained by image recognition to be mentioned later, from the memory 112, and by referring to this correlational data, converts the digital signal per unit time calculated based on the digital signal stored in the memory 112 to the biocomponent concentration. The memory 112 corresponds to the memory unit in the present invention. The biocomponent concentration converted in the microcomputer 110 is outputted to the display 114 to be displayed.

The first optical filter 122 has spectral characteristics which transmit the infrared light in the wavelength band including the wavelength absorbed by, for example, a biocomponent of the measurement target (hereinafter, referred to as measurement wavelength band). On the other hand, the second optical filter 124 has spectral characteristics different from the first optical filter 122. The second optical filter 124 has, for example, spectral characteristics which transmit the infrared light in a wavelength band including a wavelength which the measurement target biocomponent does not absorb and which other biocomponent that obstructs the measurement of the target biocomponent absorb (hereinafter, referred to as reference wavelength band). For such a biocomponent that obstructs the measurement of the target biocomponent, may be selected is a component which is present in a large amount in a living subject other than the measurement target component of the living subject.

For example, glucose shows an infrared absorption spectrum having an absorption peak in the proximity of 9.6 $\mu$m. Thus, when the measurement target (biocomponent) is glucose, the first optical filter 122 preferably has spectral characteristics that transmit the infrared light in the wavelength band including 9.6 $\mu$m.
On the other hand, protein, which is present in a large amount in a living subject, absorbs the infrared light in the proximity of 8.5 $\mu$m, and glucose does not absorb the infrared light in the proximity of 8.5 $\mu$m. Thus, the second optical filter 124 preferably has spectral characteristics that transmit the infrared light in the wavelength band including 8.5 $\mu$m.

The correlational data that shows correlations between the biocomponent concentration, and the electric signal corresponding to the intensity of the infrared light transmitted through the first optical filter 122 and the electric signal corresponding to the intensity of the infrared light transmitted through the second optical filter 324 stored in the memory 112 can be obtained, for example, by the steps below.

First, an infrared light irradiated from an eardrum of a testee having a known biocomponent is measured for a concentration (for example, a blood-sugar level). Obtained upon measurement are, the electric signal corresponding to the intensity of the infrared light in the wavelength band that the first optical filter 122 transmits, and the electric signal corresponding to the intensity of the infrared light in the wavelength band that the second optical filter 124 transmits. Such a measurement on a plurality of testees having different biocomponent concentrations provides a data set of the electric signal corresponding to the intensity of the infrared light in the wavelength band that the first optical filter 122 transmits and the electric signal corresponding to the intensity of the infrared light in the wavelength band that the second optical filter 124 transmits, and the biocomponent concentrations corresponding to these.

The measurement may also be carried out for the infrared light irradiated from the eardrum of one testee. In this case, the measurement is carried out at different timings when the biocomponent concentration (for example, a blood-sugar level) is different, for the electric signal corresponding to the intensity of the infrared light in the wavelength band that the first optical filter 122 transmits and the electric signal corresponding to the intensity of the infrared light in the wavelength band that the second optical filter 124 transmits. Carrying out the measurement at different timings provides a data set of the electric signal corresponding to the intensity of the infrared light in the wavelength band that the first optical filter 122 transmits and the electric signal corresponding to the intensity of the infrared light in the wavelength band that the second optical filter 124 transmits, and the biocomponent concentrations corresponding to these. For example, since the blood-sugar level differs according to the time elapsed after a meal and also differs depending on insulin administration, the data set of the above can be obtained at different timings.

Then, correlational data is obtained by analyzing the data set obtained in the above manner. For example, by carrying out multiple regression analysis such as PLS (Partial Least Squares Regression), and multivariate analysis by using neural network for the electric signal corresponding to the intensity of the infrared light in the wavelength band that the first optical filter 122 transmits and the electric signal corresponding to the intensity of the infrared light in the wavelength band that the second optical filter 124 transmits, and the biocomponent concentrations corresponding to these, a function showing the correlation between the electric signal corresponding to the intensity of the infrared light in the wavelength band that the first optical filter 122 transmits and the electric signal corresponding to the intensity of the infrared light in the wavelength band that the second optical filter 124 transmits, and the biocomponent concentrations corresponding to these can be obtained.

In the case when the first optical filter 122 has spectral characteristics that allows transmission of the infrared light in the measurement wavelength band and the second optical filter 124 has spectral characteristics that allows transmission of the infrared light in the reference wavelength band, the difference of the electric signal corresponding to the intensity of infrared light in the wavelength band that the first optical filter 122 transmits and the electric signal corresponding to the intensity of the infrared light in the wavelength band that the first optical filter 324 transmits may be determined to obtain correlational data illustrating the correlation between the obtained difference and the corresponding biocomponent concentrations. For example, it can be obtained by carrying out a linear regression analysis such as a least squares method.

At this time, as mentioned above, by changing the direction of the insertion probe 104 upon measurement, a plurality of correlational data that are different according to the proportion of the eardrum region can be obtained. For example, a plurality of correlational data corresponding to, for example, 40 to 90% of the eardrum region may be obtained by changing the orientation of the insertion probe 104. The larger proportion of the region is better, but even though the proportion is small, the effects of the present invention can be obtained.

A configuration for picking up an image of the eardrum 202 is described next.

The light source 140 emits a visible light for lighting the eardrum 202. The visible light emitted from the light source 140 and reflected by the first beam splitter 142 is reflected by the second beam splitter 144, and guided into the external ear canal 204 through the insertion probe 104 to light the eardrum 202.

For the light source 140, for example, visible laser such as red laser, and white LED and blue LED may be used. Among these light sources, white LED is preferable

in that the heat generation upon the emission is less compared with halogen lamp, and therefore effects on temperatures of the eardrum 202 and the external ear canal 204 are less. Instead of the light source emitting the visible light, a light source emitting a near-infrared light may also be used.

The first beam splitter 142 works to reflect a portion of the visible light, and to transmit the remaining.

The second beam splitter 144 works to reflect the visible light, and transmit the infrared light. For the second half mirror 144, a material that transmits infrared ray without absorbing, and reflects visible light is preferable. For the material of the second beam splitter 144, for example, ZnSe, CaF2, Si, and Ge may be used. The second beam splitter 144 corresponds to the light-splitting element in the present invention.

On the other hand, the visible light entered into the insertion probe 104 from the eardrum 202 through the external ear canal 204 is reflected by the second beam splitter 144, and partially transmitted through the first beam splitter 142. The visible light transmitted through the first beam splitter 142 is collected by the light collection lens 146, and reaches the image sensor 148.

For the image sensor 148, for example, CMOS and CCD are used.

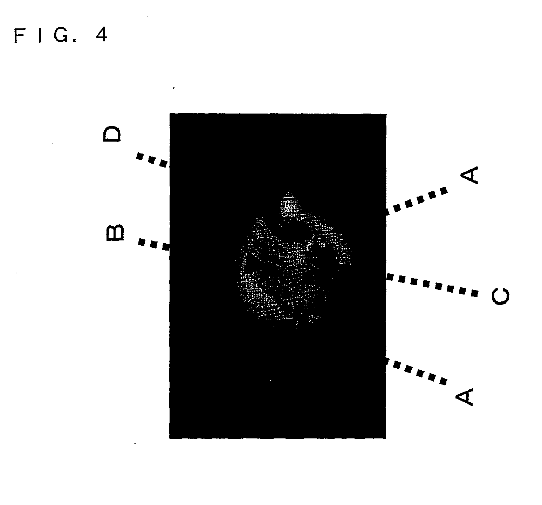

Described next is a method for recognizing the position of the eardrum 202 in the image taken by the image sensor 148. FIG. 4 is a diagram illustrating an image upon observing inside the ear cavity by using an image sensor for picking up a black and white image through an insertion probe with a diameter of 3 mm at the end portion thereof. In the diagram, the dashed, circled line shows the region where an image is picked up.

In the image, the upper left side and the lower right side (region A in FIG. 4) show the external ear canal, and the region between these (region B in FIG. 4) is the eardrum. Shown in the center (region C in FIG. 4) is a malleus contact portion, where the eardrum and the malleus are connected. Shown on the right side (region D in FIG. 4) is earwax. The position and the size of the image of the eardrum in the image picked up change depending on how deep the insertion probe is inserted, and also its orientation.

As is shown in FIG. 4, in the image picked up, the eardrum image is picked up darker than the other part. Therefore, the eardrum position can be determined by recognizing the difference in brightness (luminance) with the captured-image information detecting unit.

The information on the image obtained by the image sensor 148 is processed by using the image processing LSI 149 and the microcomputer 110 to extract the information on the region of the eardrum 202. For the image processing, for example, segmentation and extraction technique by thresholding process and labeling process as shown in the following may be used.

First, binary image processing is carried out for the image information. When an image sensor for picking up a black and white image is used, the output signal of each pixel forming the image sensor may be used as the brightness (luminance) of each pixel.

When an image sensor for picking up a color image is used, for example, a total of the red signal (R signal), the green signal (G signal), and the blue signal (B signal) outputted from each pixel may be used as the brightness (luminance) of each pixel. The average of or any one of R signal, G signal, and B signal, may also be used instead.

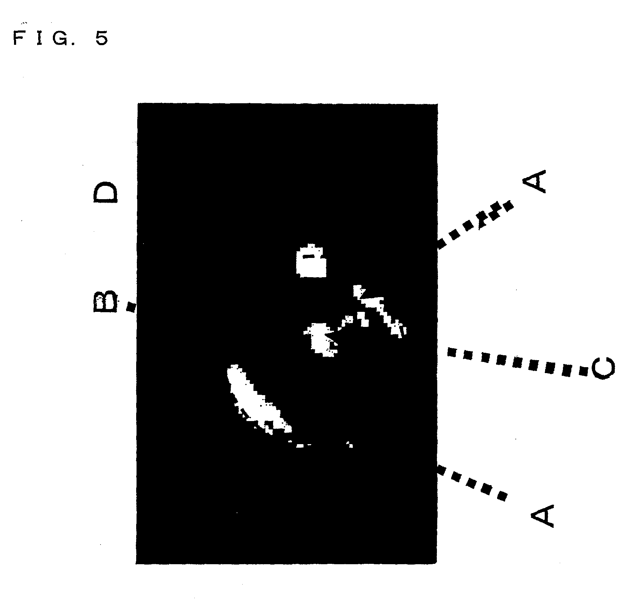

A certain reference value is set for the brightness of the pixel, and the brightness of each pixel is converted to binary numbers corresponding to black and white according to the reference value. For example, when the brightness of the pixel is equal to or more than the reference value set, white is set to that pixel, and for other pixels, black is set. Since the pixels corresponding to the portion of the eardrum 202 are darker compared with the pixels of the portions corresponding to the external ear canal 204, the malleus contact portion, and earwax, by setting the reference value to the brightness between the brightness of the pixels corresponding to the eardrum and the brightness of the pixels corresponding to the external ear canal, the malleus contact portion, and the earwax, with the above processing, the pixels corresponding to the eardrum 202 are set to black, and the pixels corresponding to the external ear canal 204 are set to white. FIG. 5 is a diagram illustrating a result of a binary image processing on the image of FIG. 4. From the figure, it is clear that the image corresponding to the eardrum is set to black.

To the image information after the above binary image processing, a labeling process, for example, is carried out. For the labeling process, for example, all the pixels in the image information after the binary image processing are scanned, and to the pixels that are set to black, the same label is added as the attributes.

Based on the above process, the region corresponding to the pixels with the label added can be recognized as the eardrum 202. The proportion of the region of the eardrum 202 relative to all the pixels in the image picked up can be obtained by computing the proportion of the pixel number equal to or less than the reference value with the microcomputer 110.

Operation of a biocomponent concentration measuring device 100 in this embodiment is described.

First, upon pressing of a power source switch 101 of a biocomponent concentration measuring device 100 by a user, a power source in a main body 102 is turned on, and the biocomponent concentration measuring device 100 is set to be in a stand-by mode for measurement. A user holds the main body 102 and inserts an insertion probe 104 into an ear cavity 200. The insertion probe 104 is a conical hollow pipe, with the diameter thereof increasing from the end portion of the insertion probe 104 to the portion thereof connecting with the main body 102. Therefore, the insertion probe 104 is formed so that the insertion probe 104 is not inserted more than the point where the external diameter of the insertion probe 104 becomes equal to the internal diameter of the ear cavity 200.

Then, upon pressing of a measurement start switch 103 of the biocomponent concentration measuring device 100 by a user while keeping the biocomponent concentration measuring device 100 at the position where the external diameter of the insertion probe 104 becomes equal to the inner diameter of the ear cavity 200, a light source 140 in the main body 102 is turned ON, and the image sensor 148 starts picking up an image.

Then, based on the above method, a step for recognizing the position of the eardrum 202 in the image taken by the image sensor 148 is carried out. When the microcomputer 110 determined that there is no image corresponding to the eardrum 202 in the image taken by the image sensor 148 as the result of the image recognition, the user is notified of an error, by a display on the display 114 with a message that the insertion direction of the insertion probe 104 is misaligned with the eardrum 202, a sound of a buzzer 158, or a sound output of a speaker (not shown).

The error may be notified to the user when the proportion of the region of the eardrum in the image picked up and computed by the microcomputer 110 is equal to or less than the threshold. When the error is notified to show that the position of the eardrum 202 cannot be recognized, the user may sift the biocomponent concentration measuring device 100 to adjust the insertion direction of the insertion probe 104. The above threshold may be determined according to the range of the proportion of the region of the eardrum in the correlational data.

The display 114, the buzzer 158, and the speaker correspond to the warning output unit of the present invention. When the microcomputer 110 determined that the position of the eardrum 202 was recognized in the image taken by the image sensor 148 as the result of the image recognition, the user can be notified of such a determination by the display 114 showing a message that the position of the eardrum 202 was recognized, a sound of the buzzer 158, or a sound output of the speaker (not shown).

When the position of the eardrum 202 is recognized, the microcomputer 110 allows the chopper 118 to start operation, which automatically starts the measurement of the infrared light irradiated from the eardrum 202. By notifying that the position of the eardrum 202 is recognized to the user, the user can know that the measurement has started, and that the biocomponent concentration measuring device 100 should not be moved and should be hold still. The speaker corresponds to the sound output unit in the present invention.

After the start of the infrared light measurement as well, the processing continues for the recognition of the position of the eardrum in the image taken by the image sensor 148. During the measurement, when the user removes the insertion probe 104 from the ear cavity 200 or changes the orientation of the insertion probe 104 largely, the microcomputer 110 detects the error in the operation of the user, by determining that there is no image corresponding to the eardrum 202 in the image taken by the image sensor 148.

Based on such a detection, the microcomputer 110 notifies the error to the user by the display 114 showing a message that the insertion direction of the insertion probe 104 is misaligned with the eardrum 202, a sound of the buzzer 158, or a sound output from the speaker (not shown). Further, the microcomputer 110 controls the chopper 118 to block the infrared light reaching the optical filter wheel 106, thereby automatically stopping the measurement. When the proportion of the region of the eardrum in the image picked up and computed by the microcomputer 110 is equal to or less than the reference value, the user may be notified of the error.

Upon being notified of the error showing that the position of the eardrum 202 cannot be recognized, the user may move the biocomponent concentration measuring device 100 to re-insert the insertion probe 104 in the ear cavity 200, or to adjust the insertion direction of the insertion probe 104, and then the measurement start switch 103 can be pressed to restart the measurement.

The microcomputer 110 blocks the infrared light reaching the optical filter wheel 106 when it determines that a certain period of time has elapsed from the measurement start based on a timer signal from the timer 156 by controlling the chopper 118. Based on this, the measurement automatically ends. At this time, the microcomputer 110 controls the display 114 or the buzzer 158 to display the message of the measurement end on the display 114, sound the buzzer 158, or output a sound to the speaker (not shown), to notify the user of the end of the measurement. Thus, the user can confirm the end of the measurement, and the insertion probe 104 can be removed out of the ear cavity 200.

The microcomputer 110 reads out, from the memory 112, the correlational data corresponding to the proportion level obtained by the above method, in the plurality of correlational data corresponding to the intensities of the infrared light transmitted through the first optical filter 122 according to the proportion of the eardrum region in the image picked up by the image sensor 148. The microcomputer 110 refers to this correlational data, and converts the electric signal outputted from the A/D converter 138 into the biocomponent concentration. The obtained biocomponent concentration is shown on the display 114.

As described above, based on the biocomponent concentration measuring device 100 of this Embodiment, with the use of the image sensor, it can be determined which organ in the ear cavity is in condition for a measurement. Further, measurement accuracy can be improved because the area of the eardrum from which the measured infrared light was irradiated can be corrected upon converting the measured signal to the biocomponent concentration.

[Embodiment 2]

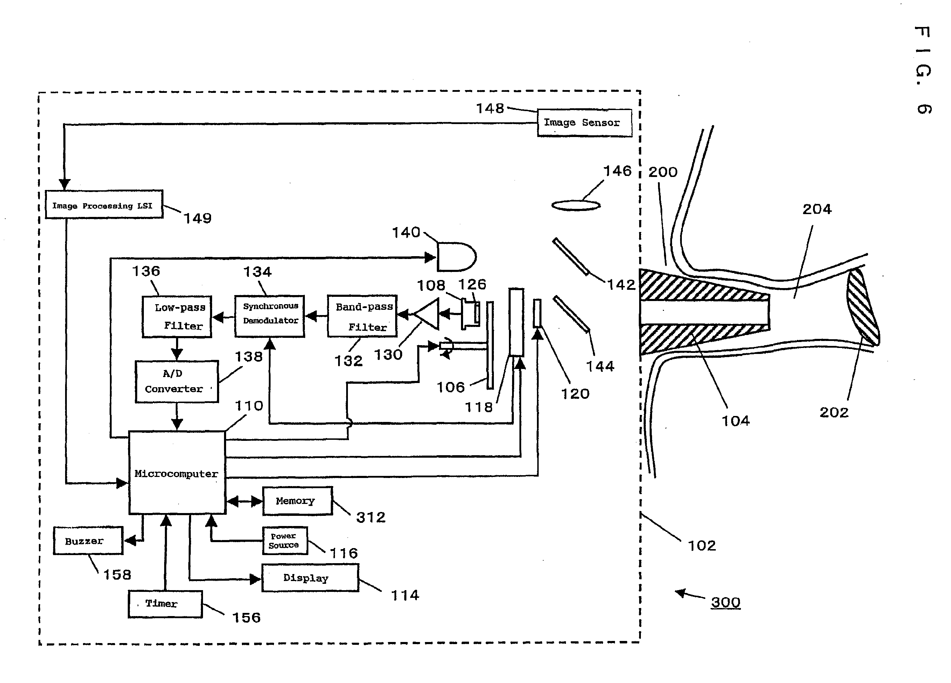

A biocomponent concentration measuring device 300 of Embodiment 2 of the present invention is described by referring to FIG. 6. FIG. 6 is a diagram illustrating a configuration of a biocomponent concentration measuring device 300 of this Embodiment.

The biocomponent concentration measuring device 300 in this Embodiment is different from the biocomponent concentration measuring device 100 of Embodiment 1 in that a liquid crystal shutter 120 is provided. Additionally, the contents of the correlational data stored in the memory 312 are different from the biocomponent concentration measuring device 100 of Embodiment 1. Other components are the same with the biocomponent concentration measuring device 100 of Embodiment 1. Therefore, the same reference numerals are used for the same components and the descriptions are omitted.

As shown in FIG. 6, the liquid crystal shutter 120 is provided between the second beam splitter 144 and the chopper 118. In the liquid crystal shutter 120, a plurality of liquid crystal cells are arranged in a matrix, and each liquid crystal cell can be controlled individually to transmit or block light based on the voltage to be applied to each liquid crystal cell.

After the microcomputer 110 recognized the image corresponding to the eardrum 202 in the image information picked up by the image sensor 148 with the image processing same as in Embodiment 1, the voltage applied to each liquid crystal cell of the liquid crystal shutter 120 is controlled, so that the liquid crystal cell to which the infrared light from the eardrum 202 enters is set to allow light transmission, and the liquid crystal cell to which the infrared light not from the eardrum 202 enters is set to block light. This allows the infrared light irradiated from the eardrum 202 to selectively enter into the infrared ray detector 108. The liquid crystal shutter 120 corresponds to the optical path control element in the present invention.

The memory 312 stores the correlational data showing correlation between the electric signal corresponding to the intensity of the infrared light transmitted through the first optical filter 122 and the electric signal corresponding to the intensity of the infrared light transmitted through the second optical filter 124, and the biocomponent concentrations. The microcomputer 110 reads out this correlational data from the memory 312, and by referring to this correlational data, converts the digital signal per unit time calculated from the digital signal stored in the memory 312 into the biocomponent concentration. The memory 312 corresponds to the memory unit of the present invention.

The correlational data showing the correlation between the electric signal corresponding to the intensity of the infrared light transmitted through the first optical filter 122 and the electric signal corresponding to the intensity of the infrared light transmitted through the second optical filter 324, and the biocomponent concentrations, which is stored in the memory 312, can be obtained, for example, by the following steps.

First, an infrared light irradiated from an eardrum of a testee having a known biocomponent is measured for a concentration (for example, a blood-sugar level). Obtained upon measurement are, the electric signal corresponding to the intensity of the infrared light in the wavelength band that the first optical filter 122 transmits, and the electric signal corresponding to the intensity of the infrared light in the wavelength band that the second optical filter 124 transmits. By carrying out this measurement on a plurality of testees having different biocomponent concentrations, a data set including the following can be obtained: the electric signal corresponding to the intensity of the infrared light in the wavelength band that the first optical filter 122 transmits, and the electric signal corresponding to the intensity of the infrared light in the wavelength band that the second optical filter 124 transmits, and the biocomponent concentrations corresponding to these signals.

Then, correlational data is obtained by analyzing the data set thus obtained. For example, by carrying out multiple regression analysis such as PLS (Partial Least Squares Regression), and multivariate analysis by using neural network for the electric signal corresponding to the intensity of the infrared light in the wavelength band that the first optical filter 122 transmits and the electric signal corresponding to the intensity of the infrared light in the wavelength band that the second optical filter 124 transmits, and the biocomponent concentrations corresponding to these, a function showing the correlation between the electric signal corresponding to the intensity of the infrared light in the wavelength band that the first optical filter 122 transmits and the electric signal corresponding to the intensity of the infrared light in the wavelength band that the second optical filter 124 transmits, and the biocomponent concentrations corresponding to these can be obtained.

In the case when the first optical filter 122 has spectral characteristics that allow transmission of the infrared light in the measurement wavelength band and the second optical filter 124 has spectral characteristics that allow transmission of the infrared light in the reference wavelength band, the difference of the electric signal corresponding to the intensity of infrared light in the wavelength band that the first optical filter 122 transmits and the electric signal corresponding to the intensity of the infrared light in the wavelength band that the first optical filter 324 transmits may be determined to obtain correlational data showing the correlation between the obtained difference and the corresponding biocomponent concentration. For example, it can be obtained by carrying out a linear regression analysis such as a least squares method.

In the following, operation of the biocomponent concentration measuring device 300 in this Embodiment is described only with regard to the operation that is different from the operation of the biocomponent concentration measuring device 100 of Embodiment 1.

When the microcomputer 110 determined that the position of the eardrum 202 was recognized in the image taken by the image sensor 148 by the same image processing as in Embodiment 1, the voltage applied to each liquid crystal cell in the liquid crystal shutter 120 is controlled so that the liquid crystal cell to which the infrared light from the eardrum 202 enters is set to transmit light, and so that the liquid crystal cell to which the infrared light not from the eardrum 202 enters is set to block light. Further, the microcomputer 110 allows the chopper 118 to operate, to start the measurement of the infrared light irradiated from the eardrum 202.

The electric signal outputted from the A/D converter 138 was corrected by the microcomputer 110 by using the proportion of the eardrum region in the image picked up with the same method as in Embodiment 1. From the memory 312, the microcomputer 110 reads out the correlational data showing the correlations between the electric signal corresponding to the intensity of the infrared light transmitted through the first optical filter 122 and the electric signal corresponding to the intensity of the infrared light transmitted through the second optical filter 124, and the biocomponent concentrations; and converts the corrected electric signal to a biocomponent concentration by referring to this correlational data. The obtained biocomponent concentration is shown on the display 114.

The method for correcting the electric signal based on the proportion of the eardrum in the image picked up can be selected based on the contents of the electric signal in the correlational data stored in the memory 312. For example, when the electric signal in the correlational data stored in the memory 312 is the signal per unit area, the measured electric signal can be corrected to the signal per unit area by using the proportion of the eardrum in the image picked up. The signal measured can be corrected as such based on the area of the eardrum from which the measured infrared light was irradiated.

In this Embodiment, the measurement can be carried out by using one correlational data. Therefore, as described in Embodiment 1 above, when a plurality of correlational data is stored in the memory 312, the correlational data with almost the same proportion of the eardrum region may be used.

As described above, based on the biocomponent concentration measuring device 100 in this Embodiment, by blocking the infrared light irradiated not from the eardrum 202 with the liquid crystal shutter 120, the infrared light irradiated from the eardrum 202 can be selectively measured, and therefore the effects from the external ear canal 204 can be removed, and highly accurate measurement can be conducted. Also, by correcting the measured signal based on the area of the eardrum from which the measured infrared light was irradiated, accuracy in measurement can be further improved.

[Embodiment 3]

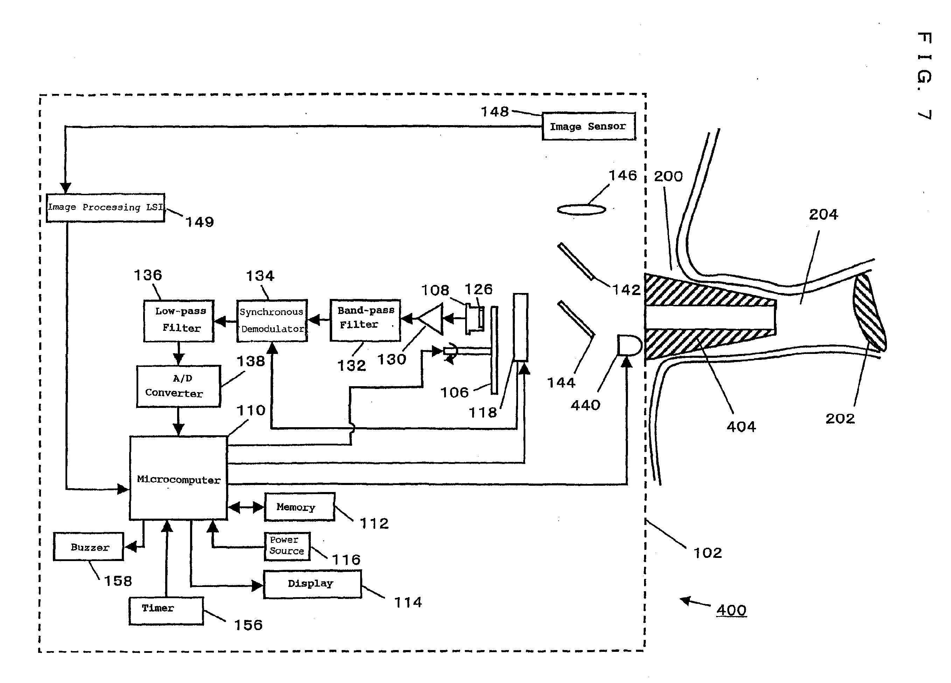

A biocomponent concentration measuring device 400 in Embodiment 3 of the present invention is described next by referring to FIG. 7. FIG. 7 is a diagram illustrating a configuration of a biocomponent concentration measuring device 400 in this Embodiment.

The biocomponent concentration measuring device 400 in this Embodiment is different from the biocomponent concentration measuring device 100 of Embodiment 1 in terms of the structure of the insertion probe 404, the position of the light source 404, and absence of the first beam splitter 142. The components other than the above is the same as that of the biocomponent concentration measuring device 100 in Embodiment 1, and therefore the same reference numerals are used and description is omitted.

An insertion probe 404 of the biocomponent concentration measuring device 400 is a hollow pipe formed by a transparent material such as acrylic resin, and a light source 404 is provided to face the connecting portion between the insertion probe 404 and the main body 102, so that light can be applied to the connecting portion between the insertion probe 404 and the main body 102.

Based on such a configuration, the light can be applied to the inner ear cavity using the main body of the insertion probe 404 as the optical guide path, not the hollow portion of the insertion probe 404. Additionally, by disposing the light source 404 to face the connecting portion between the insertion probe 404 and the main body 102, the first beam splitter 142 becomes unnecessary, and therefore with no light losses at the first beam splitter 142, the amount of light reaching the image sensor 148 can be improved.

[Embodiment 4]

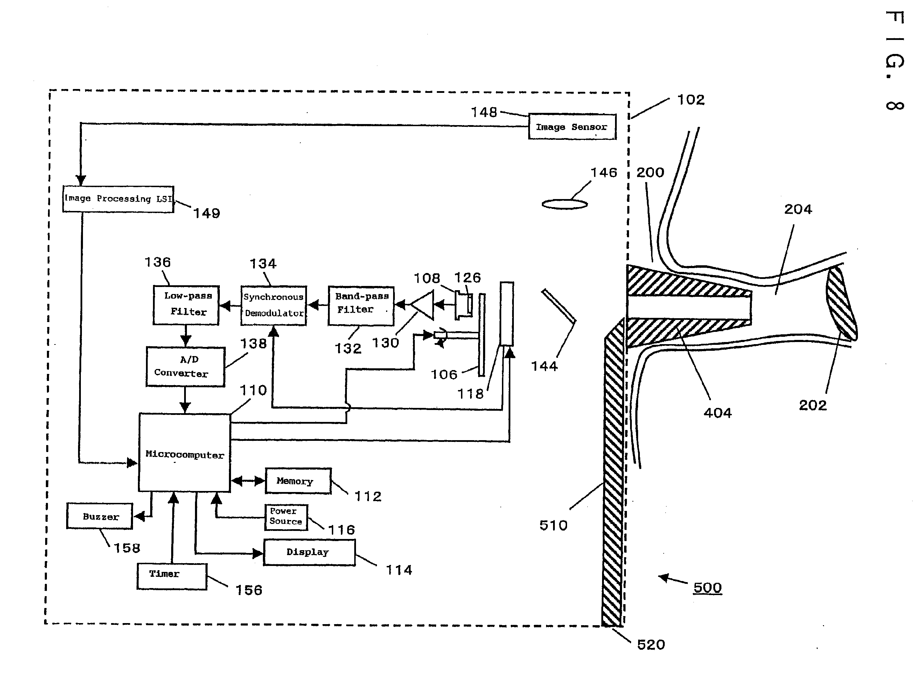

A biocomponent concentration measuring device 500 in Embodiment 4 of the present invention is described next by using FIG. 8. FIG. 8 is a diagram illustrating a configuration of a biocomponent concentration measuring device 500 in this Embodiment.

The biocomponent concentration measuring device 500 in this Embodiment is different from the biocomponent concentration measuring device 400 in Embodiment 3 in that an optical guide path 510 for letting in the light outside the biocomponent concentration measuring device 500 and guiding the light to the insertion probe 404 is provided, and the light source 440 is not used. Other components are the same as in the biocomponent concentration measuring device 400 in Embodiment 3, and therefore the same reference numerals are used to omit the description.

On the outer surface of the main body 102 of the biocomponent concentration measuring device 500, a lighting window 520 is provided. The light outside the biocomponent concentration measuring device 500 enters the optical guide path 510 through the lighting window 520, and is guided to the insertion probe 404 through the optical guide path 510. Based on such a configuration, without using a light source, the application can be made to the inner ear cavity by the light outside the biocomponent concentration measuring device 500.

For the optical guide path 510, a resin such as acrylic resin, and optical fiber may be used. Although the insertion probe 404 is formed by a transparent material in Embodiments 3 and 4, a coating of metal film such as gold or aluminum is preferably provided to the inner face of the insertion probe 404, and to the outer circumferential face of the insertion probe 404 except for the end face, to curb the entrance of the infrared light irradiated from the organ at the external ear canal through the insertion probe 404.

Additionally, in Embodiments 3 and 4, at at least a portion of an end face 404a of the insertion probe 404, a rough face that allows the light emitted from the insertion probe 404 to diffuse is preferably provided, so that uniform lighting in the inner ear cavity is possible.

[Embodiment 5]

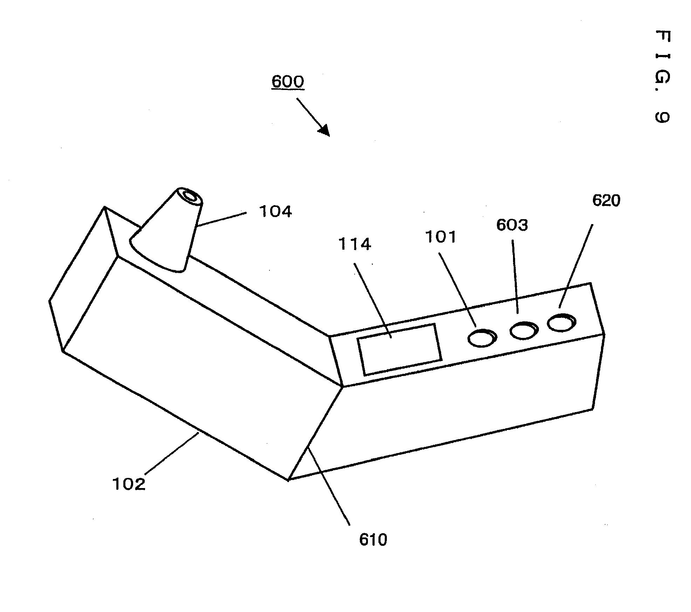

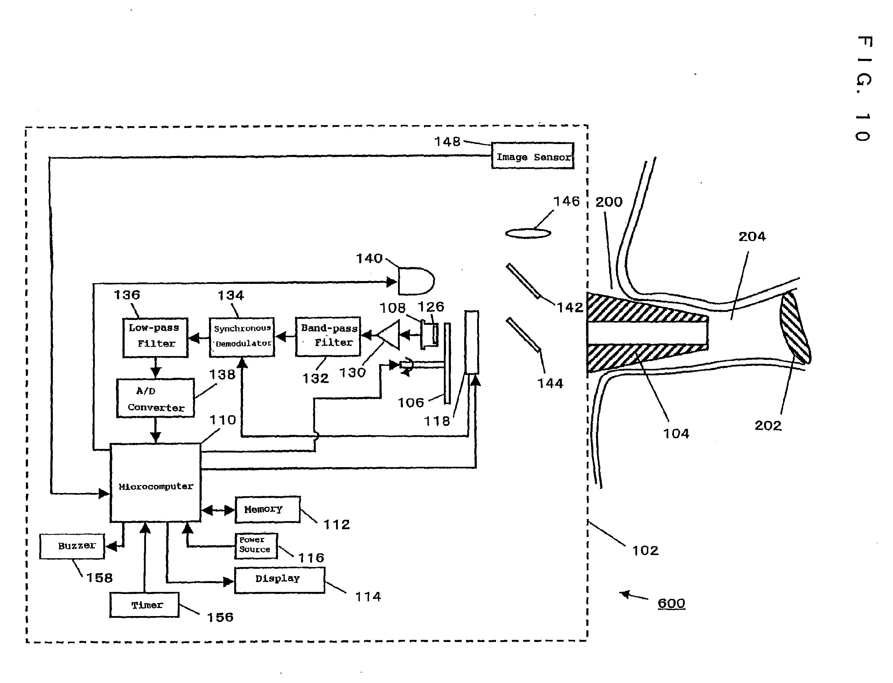

A biocomponent concentration measuring device 600 in Embodiment 5 of the present invention is described by using FIGs. 9 and 10. FIG. 9 is a perspective view illustrating an exterior of the biocomponent concentration measuring device 600 in this Embodiment, and FIG. 10 is a diagram illustrating a configuration of the biocomponent concentration measuring device 600 in this Embodiment.

As shown in FIG. 9, the biocomponent concentration measuring device 600 in this Embodiment includes a bending portion 610, and with the bending portion 610, the device is divided into two portions. In the two portions divided by the bending portion 610, an insertion probe 104 is provided on one portion, and a display 114, a power source switch 101, a measurement start switch 603, and an image-pickup start switch 620 are provided on the other portion.

In the biocomponent concentration measuring device 600, a display 114 is disposed at the position where a user can see the information on the captured-image displayed on the display 114 while the insertion probe 104 is inserted in the ear cavity of the user.

As shown in FIG. 10, the configuration inside the main body of the biocomponent concentration measuring device 600 is different from the biocomponent concentration measuring device 100 of Embodiment 1 in that the image processing LSI is not provided. Also, as in Embodiment 2, the contents of the correlational data stored in the memory 312 are different from the biocomponent concentration measuring device 100 in Embodiment 1. Other components are the same with the biocomponent concentration measuring device 100 of Embodiment 1. Therefore, the same reference numerals are used and the descriptions are omitted.

The memory 312 stores, as in Embodiment 2, the correlational data showing the correlation between the electric signal corresponding to the intensity of the infrared light transmitted through the first optical filter 122 and the electric signal corresponding to the intensity of the infrared light transmitted through the second optical filter 124, and the biocomponent concentrations. The method for obtaining this correlational data is the same as in Embodiment 2, and therefore the description is omitted.

Operation of the biocomponent concentration measuring device 600 in this Embodiment is described. First, upon pressing of a power source switch 101 of a biocomponent concentration measuring device 600 by a user, a power source in a main body 102 is turned on, and the biocomponent concentration measuring device 600 is set to be in a stand-by mode for measurement.

Then, the user holds the main body 102 and inserts the insertion probe 104 into the ear cavity 200. Then, upon pressing of the image-pickup start switch 620 of the biocomponent concentration measuring device 100 by a user while keeping the biocomponent concentration measuring device 600 at the position where the external diameter of the insertion probe 104 becomes equal to the inner diameter of the ear cavity 200, the light source 140 in the main body 102 is turned ON, and the image sensor 148 starts picking up an image.

The user visually checks if the image of the eardrum 202 is shown on the display 114 while holding the biocomponent concentration measuring device 600. When there is no image of the eardrum 202 shown on the display 114, the orientation of the insertion probe 104 in the ear cavity is adjusted manually until the image of the eardrum 202 is shown on the display 114.

After visually confirming that the image of the eardrum 202 is shown on the display 114, upon pressing the measurement start switch 603 by the user, the microcomputer 110 allows a start of the operation of the chopper 118, to start the measurement of the infrared light irradiated from the eardrum 202. The measurement start switch 603 corresponds to the instruction reception unit of the present invention.

The microcomputer 110 blocks the infrared light reaching the optical filter wheel 106 when it determines that a certain period of time has elapsed from the measurement start based on a timer signal from the timer 156 by controlling the chopper 118. Based on this, the measurement automatically ends. At this time, the microcomputer 110 controls the display 114 or the buzzer 158 to display the message of the measurement end on the display 114, sound the buzzer 158, or output a sound to the speaker (not shown), to notify the user of the end of the measurement. Thus, the user can confirm the end of the measurement, and the insertion probe 104 can be removed out of the ear cavity 200.

The microcomputer 110 reads out, from the memory 112, the correlational data showing the correlations of the electric signal corresponding to the intensities of the infrared light transmitted through the first optical filter 122 and of the infrared light transmitted through the second optical filter 124, and the biocomponent concentrations, and by referring to this correlational data, converts the electric signal outputted from the A/D converter 138 to the biocomponent concentration. The obtained biocomponent concentration is shown on the display 114.

As described above, based on the biocomponent concentration measuring device 600 of this Embodiment, since the user can confirm that the eardrum 202 is shown on the display 114 while the insertion probe 104 is being inserted in the ear cavity 200 of the user, and then enters the instruction of the measurement start via the measurement start switch 603, the effects from the external ear canal 204 can be decreased, and highly accurate measurement can be conducted.

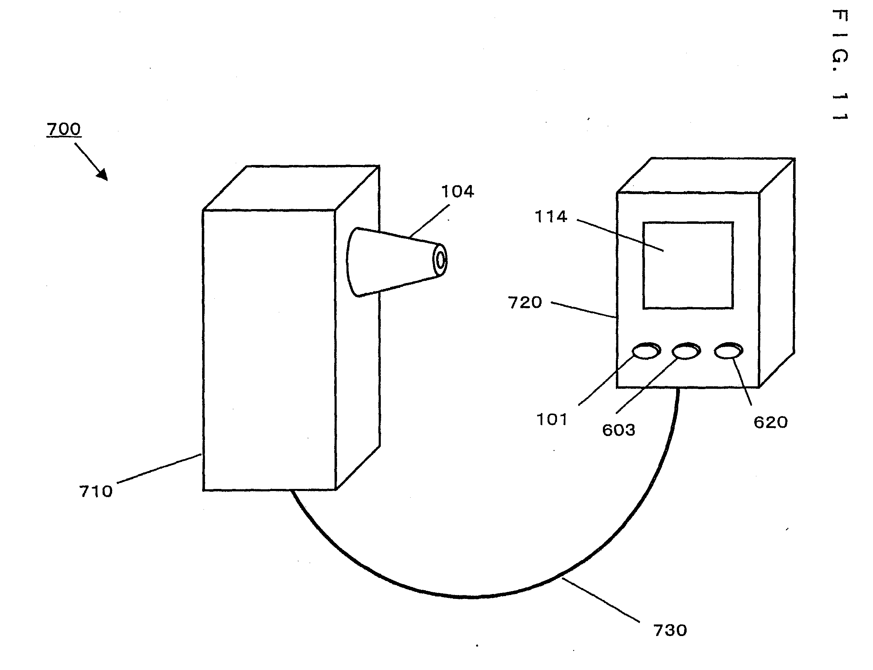

Described in this Embodiment was the biocomponent concentration measuring device 600 including the bending portion 610, and having the insertion probe 104 in one portion, and the display 114, the power source switch 101, and the measurement start switch 103 in the other portion of the portions divided into two by the bending portion 610. However, without limitation, a configuration shown in FIG. 11 can be used as well. FIG. 11 is a perspective view illustrating an exterior of a variation of a biocomponent concentration measuring device 700 in this Embodiment.

As shown in FIG. 11, the biocomponent concentration measuring device 700 of this variation includes a probe portion 710 including an insertion probe 104; and a main body portion 720 provided with a display 114, a power source switch 101, a measurement start switch 603, and an image-pickup start switch 620. The probe portion 710 and the main body portion 720 are connected by a cable 730 for transmitting the electric signal.

Although the optical filter wheel 106 in which the first optical filter 122 and the second optical filter 124 were put in the ring 123, and one infrared ray detector 108 were used in the above Embodiments, the present invention is not limited to such an embodiment. For example, instead of the optical filter wheel 106 and the infrared ray detector 108, a first infrared ray detector in which a first optical filter 122 is disposed on the infrared ray detection region, and a second infrared ray detector in which a second optical filter 124 is disposed on the infrared ray detection region may be used.

In this case, preferably, a beam splitter that transmits one of the infrared light in the wavelength band that the first optical filter 122 transmits and the infrared light in the wavelength band that the second optical filter 124 transmits, and reflects the other of the infrared light in the wavelength band that the first optical filter 122 transmits and the infrared light in the wavelength band that the second optical filter 124 transmits is further provided, and the infrared light introduced into the main body 102 by the insertion probe 104 is divided to two by wavelength with the beam splitter, so that the infrared light in the wavelength band that the first optical filter 122 transmits reaches the first infrared ray detector, and the infrared light in the wavelength band that the second optical filter 124 transmits reaches the second infrared ray detector.

Based on such a configuration, the electric signal corresponding to the intensity of the infrared light transmitted through the first optical filter 122 can be obtained from the first infrared ray detector, and the electric signal corresponding to the intensity of the infrared light transmitted through the second optical filter 124 can be obtained from the second infrared ray detector.

Although the first optical filter 122 and the second optical filter 124 were used in the above Embodiment, the present invention is not limited to such an embodiment. For example, instead of the infrared ray detector 108, an infrared ray detector having characteristics of sensitivity identical to the characteristics of wavelength of the first optical filter 122 and the second optical filter 124.

For example, by using a Quantum Well Infrared Photodetector (QWIP), the film thickness can be optimized to specifically detect the infrared light in a specific wavelength without using a dispersing filter.

Industrial Applicability

The present invention is useful in a noninvasive measurement of a biocomponent concentration, for example, a measurement of a glucose concentration without taking blood sample.

**Claims**

1. A biocomponent concentration measuring device comprising:

   an infrared ray detector for detecting an infrared light irradiated from an organ in an ear cavity;
   an image sensor for picking up an image of said organ in said ear cavity; and
   a biocomponent concentration computing unit for converting an output of said infrared ray detector into a biocomponent concentration.

2. The biocomponent concentration measuring device in accordance with claim 1, further comprising a light source for emitting a light for lighting said ear cavity,
   wherein said image sensor picks up an image of a light emitted from said light source and reflected in said organ in said ear cavity.

3. The biocomponent concentration measuring device in accordance with claim 1, further comprising a spectroscopic element for dispersing said infrared light.

4. The biocomponent concentration measuring device in accordance with claim 1, further comprising an insertion probe to be inserted in said ear cavity,
   wherein said insertion probe guides said infrared light irradiated from said organ in said ear cavity into said infrared ray detector.

5. The biocomponent concentration measuring device in accordance with claim 2, further comprising a light-splitting element for transmitting one of said light reflected in said organ in said ear cavity and said infrared light irradiated from said organ in said ear cavity, and reflects the other of said light reflected in said organ in said ear cavity and said infrared light irradiated from said organ in said ear cavity.

6. The biocomponent concentration measuring device in accordance with claim 1, further comprising a captured-image information detecting unit for detecting information on a captured-image of said eardrum from information on a captured-image obtained by said image sensor.

7. The biocomponent concentration measuring device in accordance with claim 6, wherein said biocomponent concentration computing unit corrects said biocomponent concentration based on an amount of information on said captured-image of said eardrum detected by said captured-image information detecting unit.

8. The biocomponent concentration measuring device in accordance with claim 7, wherein said captured-image information detecting unit calculates a proportion of said information on said captured-image of said eardrum in said information on said captured-image obtained by said image sensor, and
   said biocomponent concentration computing unit corrects said biocomponent concentration by using said proportion.

9. The biocomponent concentration measuring device in accordance with claim 6, further comprising a sound source for outputting a sound while said captured-image information detecting unit is detecting said information on said captured-image of said eardrum,
   wherein at least one of a volume, a frequency, and an output interval of said sound outputted from said sound source changes according to said amount of said information on said captured-image of said eardrum detected by said captured-image information detecting unit.

10. The biocomponent concentration measuring device in accordance with claim 6, further comprising a warning output unit for outputting a warning in the case when said amount of said information on said captured-image of said eardrum detected by said captured-image information detecting unit is equal to or less than a threshold.

11. The biocomponent concentration measuring device in accordance with claim 6, further comprising an optical path control element for controlling an optical path so that an infrared light irradiated from said eardrum is selectively transmitted based on said information on said captured-image of said eardrum detected by said captured-image information detecting unit.

**12.** The biocomponent concentration measuring device in accordance with claim 1, further comprising a display for showing said information on said captured-image obtained by said image sensor, and
an instruction reception unit for receiving an instruction on a measurement start of said biocomponent concentration, wherein said display is disposed at a position where a user can see said information on said captured-image shown on said display while said insertion probe is being inserted in an ear cavity of said user.


**Amended claims under Art. 19.1 PCT**

**1.** (amended) A biocomponent concentration measuring device comprising:

an infrared ray detector for detecting an infrared light irradiated from an organ in an ear cavity;
an image sensor for picking up an image of said organ in said ear cavity;
a biocomponent concentration computing unit for converting an output of said infrared ray detector into a biocomponent concentration; and
a captured-image information detecting unit for detecting information on a captured-image of an eardrum from information on a captured-image obtained by said image sensor.

**2.** The biocomponent concentration measuring device in accordance with claim 1, further comprising a light source for emitting a light for lighting said ear cavity,
wherein said image sensor picks up an image of a light emitted from said light source and reflected in said organ in said ear cavity.

**3.** The biocomponent concentration measuring device in accordance with claim 1, further comprising a spectroscopic element for dispersing said infrared light.

**4.** The biocomponent concentration measuring device in accordance with claim 1, further comprising an insertion probe to be inserted in said ear cavity,
wherein said insertion probe guides said infrared light irradiated from said organ in said ear cavity into said infrared ray detector.

**5.** The biocomponent concentration measuring device in accordance with claim 2, further comprising a light-splitting element for transmitting one of said light reflected in said organ in said ear cavity and said infrared light irradiated from said organ in said ear cavity, and reflects the other of said light reflected in said organ in said ear cavity and said infrared light irradiated from said organ in said ear cavity.

**6.** (cancelled)

**7.** (amended) The biocomponent concentration measuring device in accordance with claim 1, wherein said biocomponent concentration computing unit corrects said biocomponent concentration based on an amount of information on said captured-image of said eardrum detected by said captured-image information detecting unit.

**8.** The biocomponent concentration measuring device in accordance with claim 7, wherein said captured-image information detecting unit calculates a proportion of said information on said captured-image of said eardrum in said information on said captured-image obtained by said image sensor, and
said biocomponent concentration computing unit corrects said biocomponent concentration by using said proportion.

**9.** (amended) The biocomponent concentration measuring device in accordance with claim 1, further comprising a sound source for outputting a sound while said captured-image information detecting unit is detecting said information on said captured-image of said eardrum,
wherein at least one of a volume, a frequency, and an output interval of said sound outputted from said sound source changes according to said amount of said information on said captured-image of said eardrum detected by said captured-image information detecting unit.

**10.** (amended) The biocomponent concentration measuring device in accordance with claim 1, further comprising a warning output unit for outputting a warning in the case when said amount of said information on said captured-image of said eardrum detected by said captured-image information detecting unit is equal to or less than a threshold.

**11.** (amended) The biocomponent concentration measuring device in accordance with claim 1, further comprising an optical path control element for controlling an optical path so that an infrared light irradiated from said eardrum is selectively transmitted based on said information on said captured-image of said eardrum detected by said captured-image information detecting unit.

**12.** The biocomponent concentration measuring device in accordance with claim 1, further comprising a display for showing said information on said captured-image obtained by said image sensor, and
an instruction reception unit for receiving an instruction on a measurement start of said biocomponent concentration, wherein said display is disposed at a position where a user can see said information on said captured-image shown on said display while said insertion probe is being inserted in an ear cavity of said user.

EP 1 941 834 A1

FIG. 1

20

EP 1 941 834 A1

F I G. 2

100
102
104
106
108
110
112
114
116
118
126
130
132
134
136
138
140
142
144
146
148
149
156
158
200
202
204

Image Sensor
Image Processing LSI
Band-pass Filter
Synchronous Demodulator
Low-pass Filter
A/D Converter
Microcomputer
Memory
Power Source
Display
Timer
Buzzer

EP 1 941 834 A1

F I G. 4

FIG. 5

FIG. 6

FIG. 7

EP 1 941 834 A1

# F I G. 8

F I G. 9

F I G. 1 0

EP 1 941 834 A1

F I G.  1 1

F I G. 1 2

## INTERNATIONAL SEARCH REPORT

International application No.

PCT/JP2006/321070

A. CLASSIFICATION OF SUBJECT MATTER
*A61B5/1455*(2006.01)i, *A61B1/227*(2006.01)i, *A61B1/233*(2006.01)i

According to International Patent Classification (IPC) or to both national classification and IPC

B. FIELDS SEARCHED

Minimum documentation searched (classification system followed by classification symbols)
A61B5/1455, A61B1/227, A61B1/233

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y<br>A | JP 2005-519666 A (Welch Allyn, Inc.),<br>07 July, 2005 (07.07.05),<br>Par. Nos. [0046] to [0055]; Figs. 2 to 6 | 1-5,12<br>6-11 |
| Y<br>A | JP 2002-513604 A (Optics L.P.),<br>14 May, 2002 (14.05.02),<br>Par. Nos. [0033] to [0049]; Fig. 2 | 1-5,12<br>6-11 |
| Y<br>A | JP 2001-506164 A (BUCHERT, Janusz, M.),<br>15 May, 2001 (15.05.01),<br>Page 12, line 16 to page 15, line 3; Figs.<br>3 to 4 | 1-5,12<br>6-11 |
| Y<br>A | JP 2001-503999 A (BUCHERT, Janusz, M.),<br>27 March, 2001 (27.03.01),<br>Page 16, line 2 to page 18, 2nd line from the<br>bottom; Figs. 3 to 4 | 1-5,12<br>6-11 |

| ☒ | Further documents are listed in the continuation of Box C. | ☒ | See patent family annex. |

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>02 November, 2006 (02.11.06) | Date of mailing of the international search report<br>14 November, 2006 (14.11.06) |
|---|---|
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

International application No.

PCT/JP2006/321070

C (Continuation).    DOCUMENTS CONSIDERED TO BE RELEVANT

| Category* | Citation of document, with indication, where appropriate, of the relevant passages | Relevant to claim No. |
|---|---|---|
| A | JP 2004-532701 A  (Welch Allyn, Inc.),<br>28 October, 2004 (28.10.04),<br>Par. Nos. [0038] to [0054]; Figs. 1 to 6 | 1-12 |

Form PCT/ISA/210 (continuation of second sheet) (April 2005)

## INTERNATIONAL SEARCH REPORT
Information on patent family members

International application No.

PCT/JP2006/321070

| | | | | |
|---|---|---|---|---|
| JP 2005-519666 A | 2005.07.07 | AU 2003218069 A1 | 2003.09.22 | |
| | | CA 2478377 A1 | 2003.09.18 | |
| | | EP 1485020 A2 | 2004.12.15 | |
| | | US 2003/171655 A1 | 2003.09.11 | |
| | | WO 2003/075761 A2 | 2003.09.18 | |
| | | WO 2003/075761 A3 | 2003.11.13 | |
| JP 2002-513604 A | 2002.05.14 | AU 744758 B2 | 2002.03.07 | |
| | | AU 3968499 A | 1999.11.23 | |
| | | CA 2331697 A1 | 1999.11.11 | |
| | | EP 1075210 A1 | 2001.02.14 | |
| | | US 6002953 A | 1999.12.14 | |
| | | WO 1999/56615 A1 | 1999.11.11 | |
| JP 2001-506164 A | 2001.05.15 | AU 7491798 A | 1998.12.11 | |
| | | CA 2290957 A1 | 1998.11.26 | |
| | | CA 2290957 C | 2004.12.14 | |
| | | EA 2288 B1 | 2002.02.28 | |
| | | EP 939603 A1 | 1999.09.08 | |
| | | EP 939603 A4 | 2005.12.14 | |
| | | IL 128106 A | 2004.08.31 | |
| | | IL 128106 D0 | 1999.11.30 | |
| | | KR 2000029457 A | 2000.05.25 | |
| | | PL 331145 A1 | 1999.06.21 | |
| | | US 5823966 A | 1998.10.20 | |
| | | WO 1998/52469 A1 | 1998.11.26 | |
| JP 2001-503999 A | 2001.03.27 | AU 711156 B2 | 1999.10.07 | |
| | | AU 2599697 A | 1997.12.09 | |
| | | EA 2636 B1 | 2002.08.29 | |
| | | EP 948284 A1 | 1999.10.13 | |
| | | IL 127111 A | 2003.02.12 | |
| | | IL 127111 D0 | 1999.09.22 | |
| | | JP 3686422 B2 | 2005.08.24 | |
| | | PL 184077 B1 | 2002.08.30 | |
| | | PL 330044 A1 | 1999.04.26 | |
| | | US 5666956 A | 1997.09.16 | |
| | | WO 1997/43947 A1 | 1997.11.27 | |
| JP 2004-532701 A | 2004.10.28 | CA 2444490 A1 | 2002.12.27 | |
| | | EP 1397652 A2 | 2004.03.17 | |
| | | WO 2002/103306 A2 | 2002.12.27 | |
| | | WO 2002/103306 A3 | 2003.05.08 | |

Form PCT/ISA/210 (patent family annex) (April 2005)

34

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI05506171 B
- JP 2002513604 A
- JP 2001503999 A